# EUROPEAN PATENT SPECIFICATION

(11) **EP 1 527 178 B1**
(45) Date of publication and mention of the grant of the patent: **21.04.2010**
(21) Application number: 02756665.2
(22) Date of filing: 24.07.2002
(51) Int. Cl.: C07K 14/715, C07K 14/54, A61K 38/17, G01N 33/50, C07K 16/28, A61K 39/395, A61P 17/00

(54) **CLASS II CYTOKINE RECEPTORS AND USES THEREOF**
KLASSE-II-CYTOKINREZEPTOREN UND DEREN VERWENDUNGEN
RECEPTEURS DE LA CYTOKINE DE CLASSE II ET LEURS UTILISATIONS

(30) Priority: 26.07.2001 US 915735
(43) Date of publication of application: 04.05.2005
(73) Proprietor: Wyeth LLC, Madison, NJ 07940 (US)
(72) Inventor: DUMOUTIER, Laure, B-1200 Brussels (BE); RENAULD, Jean-Christophe, B-1200 Brussels (BE)
(74) Representative: Brasnett, Adrian Hugh
(86) International application number: PCT/US2002/023694
(87) International publication number: WO 2003/010290

(56) References cited:
- WO-A-01/46261
- WO-A-02/072607
- WO-A1-00/24758
- WO-A2-02/10393
- BLUMBERG H ET AL: "INTERLEUKIN 20: DISCOVERY, RECEPTOR IDENTIFICATION, AND ROLE IN EPIDERMAL FUNCTION" CELL, CELL PRESS, CAMBRIDGE, NA, US, vol. 104, 12 January 2001 (2001-01-12), pages 9-19, XP000996379 ISSN: 0092-8674
- RICH B E ET AL: "CYTOKINES: IL-20 - A NEW EFFECTOR IN SKIN INFLAMMATION" CURRENT BIOLOGY, CURRENT SCIENCE,, GB, vol. 11, no. 13, 10 July 2001 (2001-07-10), pages R531-R534, XP001183885 ISSN: 0960-9822
- WANG MAI ET AL: "Interleukin 24 (MDA-7/MOB-5) signals through two heterodimeric receptors, IL-22R1/IL-20R2 and IL-20R1/IL-20R2" JOURNAL OF BIOLOGICAL CHEMISTRY, AMERICAN SOCIETY OF BIOLOGICAL CHEMISTS, BALTIMORE, MD, US, vol. 277, no. 9, 1 March 2002 (2002-03-01), pages 7341-7347, XP002298036 ISSN: 0021-9258
- DUMOUTIER L. ET AL.: 'Human interleukin-10 related T cell-derived inductible factor: molecular cloningand functional characterization as an hepatocyte-stimulating factor' PNAS vol. 97, no. 18, 29 August 2000, pages 10144 - 10149, XP000946025
- DUMOUTIER L. ET AL.: 'Cloning and characterization of II-22 binding protein, a natural antagonist of II-10-related T cell-derived inducible factor/IL-22' THE JOURNAL OF IMMUNOLOGY 2001, pages 7090 - 7095, XP002206182
- KOTENKO S.V. ET AL.: 'Identification, cloning and characterization of a novel soluble receptor that binds II-22 and neutralizes its activity' THE JOURNAL OF IMMUNOLOGY 2001, pages 7096 - 7103, XP002186668
- DUMOUTIER L. ET AL.: 'Cutting edge: STAT activation by IL-19, IL-20 and Mda-7 through II-20 receptor complexes of two types' THE JOURNAL OF IMMUNOLOGY 2001, pages 3545 - 3549, XP002231865
- SHEIKH F. ET AL.: 'Cutting edge: IL-26 signals through a novel receptor complex composed of IL-20 receptor 1 and IL-10 receptor 2' THE JOURNAL OF IMMUNOLOGY 2004, pages 2006 - 2010, XP002983402

## Description

### FIELD OF THE INVENTION

This invention relates to cytokines, and the phenomenon of "receptor switching." More particularly, it relates to homologs of IL-10, and various receptors for these homologs, as well as their use.

### BACKGROUND AND PRIOR ART

Interleukin-10 ("IL-10" hereafter) is a major, anti-inflammatory cytokine, which was originally identified by Moore, et al., Annu. Rev. Immunol 19:683 (2001), as a factor which inhibited cytokine production by activated TH1 lymphocytes. Following the identification of IL-10, several additional cytokines, with varying degrees of homology to IL-10 were identified. The first of these was named "mda-7", an acronym for "melanocyte differentiation associated gene 7", because its expression was upregulated during in vitro differentiation of a melanoma cell line. See Jiang, et al., Oncogene 11:2477 (1995). This protein exhibits 22% amino acid identity with IL-10, but it was not originally recognized as a secreted protein. Expression of mda-7 is reported to provoke irreversible growth arrest of tumors via induction of apoptosis or differentiation; however, it is not clear if this effect results from a paracrine loop that involves a classic cytokine receptor pathway, or from a cytoplasmic form of the mda-7 molecule. Recently, Schaefer, et al., J. Immunol 166:5859 (2001), identified the murine orthologue of mda-7, as a TH2-specific cytokine, and named it "IL-4 induced secreted protein," or "FISP." The rat counterpart, identified by Zhang, et al., J. Biol. Chem 275:24436 (2000), is referred to as "mob5", and is expressed by intestinal epithelial cells upon ras activation. Zhang et al. have suggested that mob5 plays a role in ras oncogene-mediated neoplasia, through an autocrine loop involving a putative, ras-inducible cell surface receptor. Soo, et al., J. Cell Biochem. 74:1 (1999), have cloned the gene as a gene that is overexpressed in the skin during wound healing.

Both the *IL10* and *mda7* genes have been mapped to chromosome 1q31-32, which is a region where two other, IL-10 related genes are found, i.e., "*IL19*" and "*IL20*." IL-19 is expressed by LPS activated peripheral blood mononuclear cells, as reported by Gallagher, et al., Genes Immun 1:442 (2000). As for IL-20, its biological activities have been studied by using transgenic mice which overexpress the cytokine, where the gene is under the control of various promoters. Such mice, as reported by Blumberg, et al., Cell 104:9 (2001), are characterized by neonatal lethality, and skin abnormalities, including aberrant epidermal differentiation, which is reminiscent of psoriasis lesions in humans. Blumberg, et al., have described the IL-20 receptor complex as a heterodimer of two orphan class II cytokine receptor subunits. Specifically, "CRF2-8," for which the name "IL-20Rα" has been suggested, and "DIRS1 ", for which IL-20Rβ" has been suggested.
PCT application WO 01/46261 discloses IL-20 antagonists which are soluble forms of IL-20Rα and/or β or antibodies against IL-20Rα or β and use thereof in the treatment of diseases in which IL-20 plays a role such as atopic dermatitis and psoriasis.

Two other IL-10 cytokines, i.e., "AK155" and "IL-22" are located on human chromosome 12q15, near the IFN-γ gene. AK155 is known to be upregulated by Herpes saimiri infection of T lymphocytes. See Knappe, et al., J Virol 74:3381 (2000). The IL-22 molecule was originally described as an IL-9 inducible gene, and was referred to as "IL-TIF," for "IL-10 related T cell derived inducible factor." See Dumoutier, et al., J. Immunol 164:1814 (2000), as well as PCT Application WO 00/24758, and the U.S. priority applications referred to therein. The activities of IL-22 include the induction of the acute phase response, especially in hepatocytes and they are mediated through a heterodimeric receptor which consists of the CRF2-9/IL-22R subunit, and the β chain of the IL-10 receptor. See, e.g., Dumoutier, et al., Proc. Natl. Acad. Sci USA 97:10144 (2000); Kotenko, et al., J. Biol. Chem 276:2725 (2000); Xie, et al., J. Biol. Chem 275:31335 (2000),. Induction of the acute phase response is associated with inflammation, allergic responses, and cancer, thus suggesting that modulation of the interaction between IL-9 and IL-22 is associated with these conditions. In addition to its cellular receptor, IL-22 binds to a secreted member of the class II cytokine receptor family, referred to as "IL-22BP," or "IL-22 binding protein," which acts as a natural IL-22 antagonist. See Dumoutier, et al., J. Immunol 166:7090 (2001), Kotenko, et al., J. Immunol 166:7096 (2001),.

It will be understood from the above, that there are two classes of cytokine receptors, i.e., class I and class II. Within the class I cytokine receptors, sharing of receptor subunits is a well recognized phenomenon. Subfamilies have been defined as a result of this phenomenon, including the βc, gp130, and IL-2Rγ families. In the case of class II receptors, however, the only example of a shared receptor up to now has been the IL-10Rβ chain, which is involved in both IL-10 and IL-22 signaling. See Dumoutier, et al., Proc. Natl. Acad. Sci USA 97:10144 (2000); Kotenko, et al., J. Biol. Chem 276:2725 (2000); Xie, et al., J. Biol. Chem 275:31335 (2000). It is of interest to determine what class II cytokine receptors can and do function in connection with binding to particular cytokines. This is one feature of the invention, which will be described together with other features of the invention, in the disclosure which follows.
WO 02/072607 published on 19 September 2002 and claims a priority date of 9 March 2001. This application describes i.a. a soluble IL-22R/IL-20Rbeta heterodimeric polypeptide which comprises the extracellular domains of IL-22R and IL-20R linked together. The document also describes the use of this heterodimeric polypeptide in the treatment of psoriasis, eczema, atopic and contact dermatitis.

### DETAILED DESCRIPTION OF PREFERRED EMBODIMENTS

### EXAMPLE 1

A series of experiments were carried out in order to characterize interactions between various IL-10 homologues and receptors which belong to the class II cytokine receptor family.

To do this, coding sequences for the IL-10 homologues mda-7, IL-19, IL-20 and IL-22 were amplified via RT-PCR, using RNA from T cells which had been stimulated with anti-CD3 antibodies, using standard, art recognized techniques. The coding sequence for IL-20 was amplified from skin RNA.

The resulting cDNA molecules were then cloned into a commercially available plasmid, i.e., pCEP4, under the control of a CMV promoter. Fusion proteins, referred to as "mda-7-flag," "IL-19-flag," and "IL-22-flag" were produced. To elaborate, cDNA constructs were prepared by placing a sense primer in pCEP4 before the cloning site, as well as an antisense primer, in which the STOP codon of the cDNA was mutated, together with a nucleotide sequence coding for the first part of the flag molecule. The sequences used were:
gtccttgtag tcacctcccc cgagcttgta gaatttctg (SEQ ID NO: 1; used with mda-7);
gtccttgtag tcacctcccc cagctgagga catacttc (SEQ ID NO: 2; used with IL-19);
gtccttgtag tcacctcccc cttctgtctc ctccatcca (SEQ ID NO: 3; for IL-20);
   and
gtccttgtag tcacctcccc cgacgcaagc atttctcag (SEQ ID NO: 4; used with IL-22).

A first PCR product was prepared, and then a second PCR amplification was carried out, using the same sense primer, as well as an oligonucleotide which contained the entire flag sequence, as well as an NheI restriction site, i.e.:
actgctagct cacttgtcgt catcgtcctt gtagtcacct (SEQ ID NO: 5).

This allowed for direct cloning into the pCEP4 plasmid.

Clones which contained the cytokine-flag fusion cDNA were sequenced, using standard techniques and a commercially available automated fluorescence based system.

The fusion proteins were then produced by transient expression in HEK293-EBNA cells. To do this, cells were seeded in 6 well plates, at 8x10⁵ cells/well, one day before transfection. Transfection was then carried out using a standard lipofectamine method, following manufacturer's instructions, using 2µg of plasmid DNA.

Following this, the cells were incubated in 2 ml of medium, for four days, to maximize production of proteins.

Recombinant protein was measured, via Western blotting, using an antibody specific for the flag peptide, by combining 10µl of cell supernatant with Laemmli sample buffer, followed by boiling for 5 minutes. The proteins were separated on a precast, 14% polyacrylamide gel. Following transfer, the PVDF membrane was blocked in 5% nonfat dry milk, washed and probed with biotinylated, anti-flag antibody (25µg/ml), followed by incubation with streptavidin peroxidase (1/5000). Electrochemiluminescence was measured, using a commercially available system.

The HEK-293 cells secreted mda-7, IL-19, and IL-22-flag-fusion proteins with a heterogeneous molecular weight, ranging from 23-30 kilodaltons. The heterogeneity most likely resulted from glycosylation. The IL-20-flag-fusion protein was secreted as a single band, with a molecular weight of about 18 kilodaltons. This suggests that the protein was not glycosylated.

When the chemiluminescence signals were quantified, this showed that IL-19 and IL-22 were produced at similar levels. In contrast, IL-20 and mda-7 were produced at levels 7 fold less.

### EXAMPLE 2

The supernatants of the HEK-293 transfectants, described supra, were used to assess the interaction of the cytokines with class II cytokine receptors.

The cell line HT-29 endogenously expresses IL-22R and IL-10Rβ. Samples of these cells were transfected with construct "pGRR5." This construct, which contains 5 copies of the STAT binding site of the FcyRI gene, inserted upstream of a luciferase gene controlled by the TK promoter, can be used to monitor STAT activation by IL-22. It was assumed that, if the mechanism of action for the other IL-22 homologues was the same, a similar assay could be used to detect it.

Samples of HT-29 were electroporated (10⁷ cells in 400µl, 250V, 192Ω, 1,200µF), with 15µg ofpGRRS. The cells were seeded in 96 well plates, at 10⁵ cells/well, incubated for 5 hours at 37°C, and then were either preincubated, or not, for 1 hour with either anti-IL22R antiserum (1/500), or anti-IL-10Rβ antibodies (6µg/ml). The cells were then stimulated, for two hours, with the different supernatants. Luciferase activity was measured, using commercially available materials.

The anti-hIL22R antibodies were made by transfecting P815 mastocytoma cells with cDNA encoding human IL-22R that had been incorporated into plasmid pEF-BOS puro. To elaborate, IL-22R cDNA was amplified from a hepatoma cell line, i.e., "Hep G2," using sense primer:
ggaggactag ttgccagccc cgatgagga
   (SEQ ID NO: 6). This sense primer contains a restriction site for Spel. An antisense primer containing a restriction site for NotI, i.e.:
gtgtggcggc cgcaggcatg ggattgacag c
   (SEQ ID NO: 7) was also used. These primers facilitate direct cloning into the pEF-BOS puro expression vector. See Demoulin, et al., Mol. Cell Biol. 16:4710 (1996),

The transfectants were injected into DBA/2 mice. After the rejection of the tumors by the mice, their resulting sera contained high titers of neutralizing anti-hIL-22 antibodies. This is the antiserum referred to supra.

The HT-29 cells were responsive to IL-22, but not to the other cytokines.

### EXAMPLE 3

The work of example 2 was extended, by cotransfecting the HT-29 cells with pGRR5, and cDNA (15µg), for either IL-20Rβ or IL-20Rα. In all other ways, the experiment was exactly the same as in example 2.

When the HT-29 cells were transfected with IL-20Rβ cDNA, both mda-7 and IL-20 induced luciferase production; however, this effect was blocked completely by anti-IL-22R antiserum, indicating formation of a previously unobserved IL-20R, which contains IL-22R and IL-20Rβ.

When the HT-29 cells were transfected with cDNA for both IL-20Rα and IL-20Rβ, they became receptive to all of mda-7, IL-20, and IL-19. Further, luciferase production was not affected by the anti-IL-22R antibodies, indicating that the activity was independent of the chain.

Transfection with IL-20Rα cDNA alone did not provoke any response.

These results show that IL-20Rβ is required for the described process.

### EXAMPLE 4

A further set of experiments were then carried out, in order to characterize the different types of receptor complexes further.

HEK-293 cells express IL-10Rβ endogenously, but do not express IL-22R. Untransfected cells were admixed with supernatants, as described supra, and did not respond to any of the homologues. Samples of HEK-29 cells were seeded in 24 well plates, at 2x10⁵ cells/well one day before transfection, and were then transfected, using 100ng of pGRR5, and 500ng of plasmid encoding IL-20Rα, IL-20Rβ, or IL-22R, using the lipofectamine method referred to in example 1 and assayed with supernatants, as described supra. The cDNA for IL-20Rα was obtained by amplification of a placenta library. A cDNA fragment encoding the extracellular domain was amplified using:
gccggatcca tgcggccgct gccgctgccg
   (SEQ ID NO: 8), and
atcgctagcc atttagcctt gaactctgat g
   (SEQ ID NO: 9),
   digested with BamHI restriction endonuclease, and cloned into BamHI and EcoRV sites of commercially available, pcDEF3 vector. The resulting plasmid is referred to as "pEF2-CRF2-8EC." The PCR product encoding the transmembrane and intracellular domains was generated using:
gtggctagcc tggtatgttt tgcccat
   (SEQ ID NO: 10), and
gcgaattcgt ctggcaaaca tttattga
   (SEQ ID NO: 11), which contain NheI and EcoRI sites, respectively, to facilitate cloning into pEF2-CRF2-8EC.

The cDNA for IL-20Rβ was amplified from K562 leukemia cells, using:
ttggctagca acaatgttct aggtc
   (SEQ ID NO: 12), and
tgggcgcggc cgcaaaccta tgagat
   (SEQ ID NO: 13), which contain NheI and NotI sites, for cloning into pCEP4.

When IL-22R cDNA was transfected into the cells, IL-22 induced luciferase production and STAT-3 phosphorylation, but none of the homologues did. Cells transfected with both IL-22R and IL-20Rβ responded to IL-22, IL-20 and mda-7. Transfection with IL-20Rβ alone did not confer any responsiveness. When both IL-20Rα and IL-20Rβ were transfected, all of the mda-7, IL-19 and IL-20 provoked responses, but IL-22 did not. There was no response when IL-20Rα was used alone.

In each of these experiments, luciferase induction correlated with STAT-3 phosphorylation.

### EXAMPLE 5

The experiments set forth, supra, showed that two different complexes bound with IL-20 and mda-7. This begged the question of whether one complex would respond more favorably to one of the cytokines.

In order to examine this question, HT-29 cells were transfected, either with IL-20Rβ alone, or both IL-20Rβ and IL-20Rα, in the same way described supra. Varying dilutions (10%, 1 %, and 0.1%) of supernatants were used. Luciferase activity was measured two hours after stimulation.

When both IL-20Rα and IL-20Rβ were transfected into cells, both mda-7 and IL-20 dilutions showed a similar dose-response curve, indicating similar sensitivity to both cytokines. When only IL-20Rβ was transfected, the HT-29 cells showed better responsiveness to mda-7 at non-saturating dilutions (1% and 0.1% supernatant), indicating that this type of complex is more sensitive to mda-7.

Parallel results were obtained when experiments were repeated, using HEK-293 cells.

### EXAMPLE 6

The experiments set forth, supra, showed that IL-22R can associate both with IL-10Rβ and IL-20Rβ, suggesting that complexes of IL-20Rβ and IL-22R could mediate an IL-22 response. Experiments to test this possibility had to account for the fact that IL-10Rβ is ubiquitously expressed. Hence, the role of IL-10Rβ was assessed with an anti-IL-10Rβ antibody. HT-29 cells were transfected with pGRR5, and IL-20Rβ cDNA, as described supra. The transfectants were cultured, also as described, supra, and were then incubated with 10% supernatant from HEK-293 cells that had either been transfected with IL-22 cDNA, or had been mock transfected. Luciferase activity was monitored 2 hours later.

The results showed that the anti-IL-10Rβ antibody blocked IL-22 activity both in control cells, and in cells transfected with IL-20Rβ cDNA. This indicates that IL-20Rβ cannot substitute for IL-10Rβ, when this is not accessible to IL-22.

In related experiments, the antibody did not affect activity of mda-7 or IL-20, in the same cells.

### EXAMPLE 7

Work by Dumoutier, et al., J. Immunol 166:7090(2001) and Kotenko, et al., J. Immunol 166:7096(2001), showed that IL-22BP binds to IL-22. There have not been any other reports on whether this molecule binds to other cytokines. As it exhibits the same degree of homology with the extracellular domains of IL-22R and IL-20Rα, it was suggested that IL-22BP could also bind IL-20, or other molecules.

To test this, first maleic anhydride activated polystyrene plates were coated with biotinylated anti-flag antibody by incubating the plates, overnight, at 4°C, with 12.5µg/ml of the antibody in PBS. Plates were washed in PBS buffer containing Tween 20 (0.01%), and then blocked with bovine serum albumin (1% in PBS) for two hours, followed by incubation for 2 hours with 50µ1 of cytokine flag fusion proteins, by way of HEK-293 supernatants, produced as described supra. After washing, 10% supernatants of cells that had been transfected with vectors that produced IL-22BP-Ig were added. See Dumoutier, et al., supra, incorporated by reference, for information on production of this fusion protein. The supernatant was removed after 2 hours, and any bound IL-22BP-Ig was detected by adding anti-mouse IgG3 polyclonal antibodies, coupled to peroxidase. The enzymatic activity was then determined by adding 3, 3', 5, 5' tetramethylbenzidine. The reaction was stopped by adding 20µl of H₂SO₄ (2M). Reactivity was determined by reading absorbance values, at 450nm.

Only IL-22 was able to bind IL-22BP-Ig. No homologues showed any activity.

In related experiments, the inhibitory effect of IL-10 homologues on the binding of IL-22BP to IL-22 was determined. To do this, plates, as described supra, were coated with recombinant human IL-22, as described by Dumoutier, et al., Proc. Natl. Acad. Sci USA 97:10144(2000),. The plates were then contacted with IL-22BP-Ig (10% supernatant) that had been preincubated with the various IL-10 homologues, 2 hours before contact with the plates. Any bound IL-22BP-Ig was determined in the same manner described in the first set of experiments. The interaction between IL-22 and IL-22BP was detectable with the anti-Ig antibody, and only IL-22 supernatants were able to block the IL-22BP binding.

The foregoing examples describe, inter alia, a new cytokine receptor complex, which comprises an IL-22R molecule, and an IL-20Rβ molecule. As is shown, supra, this receptor complex facilitates the binding of cytokines such as mda-7 and IL-20. The examples also show that complexes containing IL-20Rα and IL-20Rβ bind these cytokines, as well as IL-19. Both complexes can serve, inter alia, as agents for determining whether or not their respective binding partners are present in a sample. For example, the present application shows that IL-20Rα and IL20Rβ form a receptor for mda-7, IL-19, and IL-20. Complexes of IL-22R and IL-20Rβ form a receptor for mda-7 and IL-20, but not IL-19. Hence, by using various combinations of these receptors, one can determine which IL-10 related cytokines are or are not present in a sample. Receptors such as the complex of IL-22R and IL-20 Rβ, or variants described below, can be used in assaying for agents and substances that, by affecting the association or interaction between IL-22R and IL-20Rβ, modulate IL-20 and/or mda-7 function *in vivo*. Similarly, substances that, by affecting association or interaction between IL-20Rα and IL-20Rβ or variants thereof modulate IL-19, IL-20 and mda-7 function *in vivo* can be determined. Formats that may be used in such assays are described in detail below, but in brief may comprise determining binding between component polypeptide chains in the presence or absence of a test substance, and/or determining ability of a receptor of the invention to bind a ligand, such as a cytokine, in the presence or absence of a test substance, and/or determining ability of a test substance to modulate a biological or cellular function or activity in which the receptor plays a role, which function or activity may be affected by a binding partner as described supra, selected from those shown to bind the receptor or may involve an activity downstream of the receptor in a biological pathway. Assay methods that involve determination of binding between components of a receptor complex and the effect of a test substance on such binding need not necessarily utilize full-length polypeptide chains. For instance, fragments that retain the ability to bind the companion polypeptide chain component of the receptor may be employed. Indeed, as discussed further below, fragments of the polypeptides themselves represent a category of putative inhibitors, that may be used to interfere with binding between receptor polypeptide components.

Further, such an isolated receptor or a fragment that retains ability to bind a cytokine, may be used in determining the presence in a sample or test material of a cytokine to which it binds, e.g. IL-20 or mda-7 for a receptor comprising IL-22R and IL-20Rβ and IL-19 or mda-7 for a receptor comprising IL-20Rα/IL-20Rβ This too is discussed further below.

A receptor complex for methods or uses herein may be provided in isolated form by a method of purification from cells that endogenously produce the relevant component polypeptide chains. More usually, however, and more preferably, the receptor complex is provided by means of recombinant production by expression from encoding nucleic acid molecules in a suitable expression system, followed by purification, e.g., using suitable binding antibody molecule or the ligands themselves as well as via the use of fusion proteins, such as fusion proteins comprising a part of the ligands sufficient to bind to receptor.

Isolated nucleic acid molecules encoding the receptor polypeptide chains utilized here in are available in the art.

Such nucleic acid molecules may be used to provide vector constructs which encode the receptor polypeptide chains identified herein.

Receptors that comprise combinations of polypeptide chains as identified herein may be made by co-expression from nucleic acid molecules encoding both chains, or may be made by combination of polypeptide chains produced separately and then allowed to associate. The receptor molecules may be glycosylated or non-glycosylated, sulfated or non-sulfated, as well as in forms involving other post translational modifications such as, but not being limited to, acetylation, acylation, phosphorylation, palmitoylation, ubiquitination, ADP-ribosylation, hydroxylation, glucosylphosphatidylinositide addition, oxidation, reduction and so forth.

The person skilled in the art may employ any available technique for manipulation of coding sequences and recombinant production of encoded polypeptides and peptides to manufacture the desired molecules

Generally, nucleic acid molecules employed to produce receptors and polypeptide chains or fragments thereof described herein, for example nucleic acid molecules with separate sequences encoding a novel combination of receptor polypeptide chains as identified herein, are provided as isolates, in isolated and/or purified form, or free or substantially free of material with which they are naturally associated, such as molecules that are free or substantially free of nucleic acids flanking the gene in the (e.g. human) genome, except possibly one or more regulatory sequence(s) for expression. Nucleic acid molecules may be wholly or partially synthetic and include genomic DNA, cDNA and RNA.

Nucleic acid molecules encoding the peptides or polypeptides described herein may be readily prepared by the skilled person using the information and references contained herein and techniques known in the art (for example, see Sambrook and Russell "Molecular Cloning, A Laboratory Manual", Third Edition, Cold Spring Harbor Laboratory Press, 2001, and Ausubel et al, Current Protocols in Molecular Biology, John Wiley and Sons, 1992, or later edition thereof). Nucleic acid molecules which encode peptide fragments may be generated and used in any suitable way known to those of skill in the art, including taking encoding molecules, identifying suitable restriction enzyme recognition sites either side of the portion to be expressed, and cutting our said portion from the molecules. The portion may then be operably linked to a suitable promoter in a standard commercially available expression system. Another recombinant approach is to amplify the relevant portion of the DNA with suitable PCR primers.

Modifications to the nucleic acid molecules may be made, e.g. using site directed mutagenesis, to lead to the production of modified polypeptides, e.g. an allele or mutant form of a polypeptide, or to take account of codon preference in the host cells used to express the nucleic acid.

A polypeptide which is an amino acid sequence variant, allelic isoform, derivative or mutant of a defined wild-type amino acid sequence may be employed in the methods described herein, provided it has the ability to bind a partner polypeptide chain and/or ligand. A variant, allelic isoform, derivative or mutant may comprise an amino acid sequence which shares greater than 60% sequence identify with the known sequences, greater than about 70%, greater than about 80%, greater than about 90% or greater than about 95%. The sequence may share greater than about 70% similarity, greater than about 80% similarity, greater than about 90% similarity or greater than about 95% similarity with the amino acid sequence described herein. Sequence comparison may be made over the full-length of the relevant sequence shown herein, or may more preferably be over the length of a specific protein domain.

Amino acid "identity" and "similarity" in relation to sequences are terms familiar to those skilled in the art in accordance with established principles and may be determined for example using the algorithm GAP (Genetics Computer Group, Madison, WI). GAP uses the Needleman and Wunsch algorithm to align two complete sequences that maximizes the number of matches and minimizes the number of gaps. Generally, the default parameters are used, with a gap creation penalty = 12 and gap extension penalty = 4. Use of GAP may be preferred but other algorithms may be used, e.g. BLAST (which uses the method of Altschul et al. (199) J. Mol. Biol. 215: 405-410), FASTA (which uses the method of Pearson and Lipman (1988) PNAS USA 85: 2444-2448), or the Smith-Waterman algorithm (Smith and Waterman (1981) J. Mol Biol. 147: 195-197), generally employing default parameters.

At the nucleic acid level, relatedness of sequence may be determined by means of selective hybridization between molecules under stringent conditions.

For example, hybridizations may be performed, according to the method of Sambrook using a hybridization solution comprising: 5X SSC (wherein "SSC" = 0.15 M sodium chloride; 0.15 M sodium citrate; pH7), 5X Denhardt's reagent, 0.5-1.0% SDS, 100 µg/ml denatured, fragmented salmon sperm DNA, 0.05% sodium pyrophosphate and up to 50% formamide. Hybridization is carried out at 37-42 °C for at least six hours. Following hybridization, filters are washed as follows: (1) 5 minutes at room temperature in 2X SSC and 1% SDS; (2) 15 minutes at room temperature in 2X SSC and 0.1 % SDS; (3) 30 minutes - 1 hour at 37 °C in 1X SSC and 1% SDS; (4) 2 hours at 42-65 °C in 1X SSC and 1% SDS, changing the solution every 30 minutes.

One common formula for calculating the stringency conditions required to achieve hybridization between nucleic acid molecules of a specified sequence homology is (Sambrook): Tₘ = 81.5 °C + 16.6 Log [Na+] + 0.41 (% G+C) - 0.63 (% formamide) - 600/#bp in duplex. As an illustration of this formula, using [Na+] = [0.368] and 50-% formamide, with GC content of 42% and an average probe size of 200 bases, the Tₘ is 57 °C. The Tₘ of a DNA duplex decreases by 1-1.5 °C with every 1% decrease in homology. Thus, targets with greater than about 75% sequence identify would be observed using a hybridization temperature of 42 °C.

Other suitable conditions include, e.g. for detection of sequences that are about 80-90% identical, hybridization overnight at 42 °C in 0.25M Na₂BPO₄, pH 7.2, 6.5% SDS, 10% dextran sulfate and a final wash at 55 °C in 0.1X SSC, 0.1% SDS. For detection of sequences that are greater than about 90% identical, suitable conditions include hybridization overnight at 65°C in 0.25M Na₂HPO₄, pH 7.2, 6.5% SDS, 10% dextran sulfate and a final wash at 60°C in 0.1X SSC, 0.1% SDS.

In order to obtain expression of the nucleic acid molecules for the uses of the invention, the sequences may be incorporated in a vector having one or more control sequences operably linked to the nucleic acid to control its expression. Vectors may be chosen or constructed. They may contain appropriate regulatory sequences, including promoter sequences, terminator fragments, polyadenylation sequences, enhancer sequences, marker genes and other sequences as appropriate, e.g. nucleic acid sequences so that the polypeptide or peptide is produced as a fusion and/or nucleic acid encoding secretion signals so that the peptide produced in the host cell is secreted from the cell. Vectors may be plasmids, viral e.g. phage, or phagemid, as appropriate. Encoded product may then be obtained by transforming the vectors into host cells in which the vector is functional, culturing the host cells so that the product is produced and recovering the product from the host cells or the surrounding medium. The method includes introducing a nucleic acid molecule encoding the combination of receptor polypeptide chains as disclosed, into a host cell. The introduction, which may (particularly for in vitro introduction) be generally referred to without limitation as "transformation" or "transfection", may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage. As an alternative, direct injection of the nucleic acid could be employed. Marker genes such as antibiotic resistance or sensitivity genes may be used in identifying clones containing nucleic acid of interest, as is well known in the art.

The introduction may be followed by causing or allowing expression of the nucleic acid molecule, e.g. by culturing host cells (which may include cells actually transformed although more likely the cells will be descendants of the transformed cells) under conditions for expression of the nucleic acid molecule, so that the encoded product is produced. If the product is expressed coupled to an appropriate signal peptide, such as a leader peptide, it may be secreted from the cell into the culture medium. Following production by expression, a product may be isolated and/or purified from the host cell and/or culture medium, as the case may be, and subsequently used as desired, e.g. in an assay or test as disclosed herein.

In light of the above, also described is a method of making a receptor complex as disclosed, the method comprising causing or allowing expression from nucleic acid molecules encoding the receptor polypeptide chains, combining the chains to form the receptor complex and purifying the complex. The expression of two chains of the complex may involve co-expression within a single expression system, e.g. host cell, or the two chains may be produced separately, subjected to an optional intermediate step of purification, then combined to form the receptor, which may then itself be purified.

Expression may conveniently be achieved by growing a host cell containing the nucleic acid molecule in culture under appropriate conditions which cause or allow expression of the peptide. Note however that expression may also be carried out in *in vitro* systems, e.g. reticulocyte lysate.

Following production of receptor complexes, e.g., comprising a combination of polypeptide chains as identified herein, it may be tested for ability to bind a cytokine. Thus, for example a receptor comprising IL-22R and IL-20Rβ may be tested for binding to one or more of IL-20 and mda-7; and a receptor comprising IL-20Rβ and IL-20Rα may be tested for binding to one or more of IL-20, IL-19 and mda-7.

Also described is a host cell containing heterologous nucleic acid molecules encoding receptor polypeptide chains as disclosed herein. The nucleic acid molecules may be integrated into the genome (e.g. chromosome) of the host cell. Integration may be promoted by inclusion of sequences which promote recombination with the genome, in accordance with standard techniques. The nucleic acid may be on an extra-chromosomal vector within the cell, or otherwise identifiably heterologous or foreign to the cell.

Thus, a host cell containing a nucleic acid molecule, e.g. as a result of introduction of the nucleic acid into the cell or into an ancestor of the cell, may express a receptor described herein, which may render the cell responsive to one or more cytokines as disclosed.

Assays employing receptors, polypeptide chains and fragments thereof, as described herein, take any of a variety of formats.

In the following discussion, for convenience reference is made to a "first polypeptide chain" and a "second polypeptide chain" of receptors of the invention. In different embodiments this will be understood to refer to the respective polypeptide chains of the receptor combination identified herein, i.e. IL-22R/IL-20Rβ and so on.

An assay method for an agent which modulates interaction between first and second polypeptide chains of a receptor of the invention may comprise:
(a) bringing into contact a first polypeptide chain of the receptor or a fragment of the first polypeptide chain, which chain or fragment binds a second polypeptide chain of the receptor; a second polypeptide chain of the receptor or a fragment of the second polypeptide chain, which chain or fragments binds the first polypeptide chain; and a test compound; and
(b) determining interaction or binding between the first and second polypeptide chains or fragments in the presence of the test compound, compared with interaction or binding between the first and second polypeptide chains or fragments in the absence of the test compound.

A test compound or agent which reduces or inhibits interaction or binding between the first and second polypeptide chains of the receptor may be identified and/or obtained under conditions in which, in the absence of the test compound being an inhibitor, the first and second substances interact or bind.

A test compound or agent which increases or potentiates interaction between the first and second polypeptide chains of the receptor may be identified using conditions which, in the absence of a positively-testing agent, prevent or impair the polypeptide chains from interacting or binding.

Also described is an assay method for a substance able to interact with the relevant region of a first polypeptide chain of a receptor of the invention that binds a second polypeptide chain of the receptor, or the relevant region of a second polypeptide chain of a receptor of the invention that binds a first polypeptide chain of the receptor, as the case may be, the method comprising:
(a) bringing into contact a first polypeptide chain of a receptor of the invention or fragment thereof that binds a second polypeptide chain of the receptor; or said second polypeptide chain or a fragment thereof that binds said first polypeptide chain, and a test compound; and
(b) determining interaction or binding between said first or second polypeptide chain or fragment thereof and the test compound.

A test compound found to interact with or bind to the relevant portion of the first or second polypeptide chain may be tested for ability to modulate, e.g. disrupt or interfere with, binding between the first and second polypeptide chains, or to modulate an activity mediated by the receptor herein described.

In a further aspect the present invention provides an assay method for an agent that modulates binding of a receptor herein described to a ligand, e.g. cytokine, the method comprising:
contacting a receptor herein described with a ligand and a test substance, under conditions in which in the absence of the test substance being an inhibitor of binding of the ligand to the receptor the ligand binds the receptor, and
determining binding of the ligand to the receptor.

Modulation of binding identifies the test substance as an agent that modulates binding of the receptor to the ligand.

The ligand may be a cytokine selected from those identified herein as being a modulator of the receptor herein described.

Also disclosed is an assay method for an agent that modulates biological activity of a receptor herein described, the method comprising providing the receptor in a host cell, and determining activity of the receptor in the presence and absence of a test substance.

Activity of the receptor may be determined by means of responsiveness of the cell to treatment with a molecule that is agonistic or antagonistic for the receptor. For example, a cell expressing a receptor of the invention may be treated with a cytokine (e.g. IL-20 for a receptor comprising IL-22R and IL-20Rβ).

In different assays herein described, interaction and/or binding and/or activity of components in the presence of a test compound or substance may be compared with the interaction and/or binding and/or activity in comparable reaction medium and conditions in the absence of a test compound. A test compound able to modulate the interaction and/or activity may be identified. The skilled person is well aware of appropriate experimental controls to perform when conducting assay methods.

Compounds which may be screened may be natural or synthetic chemical compounds used in drug screening programs. Extracts of plants, microbes or other organisms, which contain several characterized or uncharacterized components may also be used.

Also, combinatorial library technology provides an efficient way of testing a potentially vast number of different substances for ability to modulate an interaction. Such libraries and their use are known in the art, for all manner of natural products, small molecules and peptides, among others.

The use of peptide libraries may be preferred in certain circumstances. The potential for binding between polypeptide chains of receptors herein described to be inhibited by means of peptide fragments of the polypeptide chains has been mentioned already. Such peptide fragments may consist of for example 10-40 amino acids, e.g. about 10, about 20, about 30 or about 40 amino acids, or about 10-20, 20-30 or 30-40 amino acids. These may be synthesized recombinantly, chemically or synthetically using available techniques.

A further class of inhibitors of binding of a receptor of the invention to a ligand of the invention includes receptors modified to abolish effector function while retaining ability to bind ligand, e.g. a cytokine. Cytokine receptors usually contain 4 domains: the first one is the signal peptide, a 15-30 (usually about 20) amino acid region, mainly hydrophobic, which is cleaved after transfer of the protein to the endoplasmic reticulum (and is thus not present in the mature protein at the cell surface). The second domain is the extracellular domain. For the receptors of the inventions, this domain is about 200 amino acids long and shows some homology between the receptors (approx. 20-30% amino acid identity). The third domain is the transmembrane domain, a stretch of approx. 20 hydrophobic amino acids. Finally, the intracellular domain has variable size in cytokine receptors (20-3000AA), and shows little homology between receptors of the same family. Programs to predict signal peptides and transmembrane domains are available on the web (see e.g. www.cbs.dtu.dk/services/TMHMM-2.0/: for references see S. Moller, M.D.R. Croning, R. Apweiler. Evaluation of methods for the prediction of membrane spanning regions, Bioinformatics, 17(7):646-653, July 2001, or A. Krogh, B. Larsson, G. von Heijne, and E.L.L. Sonnhammer. Predicting transmembrane protein topology with a hidden Markov model: Application to complete genomes. Journal of Molecular Biology, 305(3):567-580, January 2001.). This helps define extracellular domains which might be used as inhibitors. Ways to generate such soluble receptors might be by inserting a stop condon before the transmembrane domain. Alternatively, the extracellular domain can be produced in fusion with other proteins such as peptides used to make purification easier (e.g., poly-his peptides which bind to metal ions, such as Ni²⁺), immunoglobulin Fc fragments, which allow dimerization of the receptor (this increases avidity for the ligand). Also, co-expression in a cell of fusion constructs with Ig for the 2 receptor chains leads to the production of heterodimers, reconstituting type 1 or type 2 IL-20R complexes, linked through the immunoglobulin Fc fragment. Two extracellular domains could also be produced as a single chain by including a 'space peptide' between the sequences of the first and the second chain. Such a protein might fold as the complex of the original separate proteins.

In any assay method according to the invention, the amount of test substance or compound which may be added to an assay of the invention will normally be determined by trial and error depending upon the type of compound used. Typically, from about 0.001 nM to 1mM or more concentrations of putative inhibitor compound may be used, for example from 0.01 nM to 10mM, e.g. 0.1 to 50 µM, such as about 10 µM. Even a molecule which has a weak effect may be a useful lead compound for further investigation and development.

A screening or assay method may include purifying and/or isolating a test compound and/or substance of interest from a mixture or extract, i.e., reducing the content of at least one component of the mixture or extract, e.g. a component with which the test substance is naturally associated. The screening or assay method may include determining the ability of one or more fractions of a test mixture or extract to bond to a polypeptide chain or receptor of the invention or determining the ability of one or more fractions of a test mixture or extract to bind to or affect the activity of a receptor.

The purification and/or isolation of the molecules may employ any method known to those skilled in the art, for instance using an antibody molecule.

In addition to their use in purifying polypeptide chains and receptors herein described, antibody molecules represent themselves a further class of potential modulators of receptor function.

Antibodies directed to the site of binding in one polypeptide chain of a receptor for another polypeptide chain of the receptor, or directed to a site of binding of the receptor for ligand, form another class of putative modulators of receptor activity. Candidate inhibitor and/or agonist antibodies may be characterized and their binding regions determined to provide single chain antibodies and fragments thereof which are responsible for disrupting the binding.

Antibodies may be obtained using techniques which are standard in the art. Methods of producing antibodies include immunising a mammal (e.g. mouse, rat, rabbit, horse, goat, sheep or monkey) with the relevant receptor, polypeptide chain or a peptide fragment thereof. Antibodies may be obtained from immunised animals using any of a variety of techniques known in the art, and screened, preferably using binding of antibody to antigen of interest. For instance, Western blotting techniques or immunoprecipitation may be used (Armitage et al., 1992, Nature 357: 80-82). Isolation of antibodies and/or antibody-producing cells from an animal may be accompanied by a step of sacrificing the animal.

As an alternative or supplement to immunizing a mammal with a peptide, an antibody may be obtained from a recombinantly produced library of expressed immunoglobulin variable domains, e.g. using lambda bacteriophage or filamentous bacteriophage which display functional immunoglobulin binding domains on their surfaces; for instances see PCT Application W092/0147, incorporated by reference. Such methodologies are especially preferred in preparing humanized, chimerized, or other forms of hybrid antibodies, or fragments of whole antibodies that are hybrids of, e.g, human and murine forms, human and rat forms, human and rabbit forms, or other animals which are not rodents, including but not being limited to goats, sheeps, chimpanzee, ape, and other animals.

Antibody molecules useful in accordance with the present invention may be modified in a number of ways. Indeed the term "antibody molecule" should be construed as covering covers antibody fragments and derivatives able to bind antigen. Example antibody fragments, capable of binding an antigen or other binding partner are the Fab fragment consisting of the VL, VH, C1 and CH1 domains; the Fd fragment consisting of the VH and CH1 domains; the Fv fragment consisting of the VL and VH domains of a single are of an antibody; the dAb fragment which consists of the VH domain; isolated CDR regions and F(ab')2 fragments, a bivalent fragment including two Fab fragment linked by a disulphide bridge at the hinge region. Single chain Fv fragments are also included.

Hybridomas capable of producing antibody with desired binding characteristics are within the scope of the presents invention, as are host cells, eukaryotic or prokaryotic, containing nucleic acid encoding antibodies (including antibody fragments) and capable of their expression. The invention also provides methods of production of the antibodies including growing a cell capable of producing the antibody under conditions in which the antibody is produced, and preferably secreted.

The reactivities of antibodies on a sample may be determined by any appropriate means. Tagging with individual reporter molecules is one possibility. The reporter molecules may directly or indirectly generate detectable, and preferably measurable, signals. The linkage of reporter molecules may be directly or indirectly, covalently, e.g. via a peptide bond or non-covalently. Linkage via a peptide bond may be as a result of recombinant expression of a gene fusion encoding antibody and reporter molecule. The mode of determining binding is not a feature of the present invention and those skilled in the art are able to choose a suitable mode according to their preference and general knowledge.

Antibodies may also be used in purifying and/or isolating a polypeptide chain or receptor of the invention, or a peptide fragment, for instance following production of a peptide by expression from encoding nucleic acid. Antibodies may be useful in a therapeutic context (which may include prophylaxis) to disrupt binding of polypeptide chains of a receptor or binding of receptor to ligand with a view to inhibiting the relevant biological function or activity. Antibodies can for instance be micro-injected into cells, e.g. at a tumor site.

Irrespective of the nature of the test compounds being tested in an assay method of the invention, interaction or binding between substances, e.g. polypeptide chains of a receptor herein, may be determined by any number of techniques available in the art, qualitative or quantitative. They include techniques such as radioimmunoassay, co-immunoprecipitation, scintillation proximity assay and ELISA methods.

Binding of one component to another may be studied by labeling either one with a detectable label and bringing it into contact with the other which may have been immobilized on a solid support. Suitable detectable labels, especially for peptidyl substances include ³⁵S-methionine which may be incorporated into recombinantly produced peptides and polypeptides. Recombinantly produced peptides and polypeptides may also be expressed as fusion proteins containing an epitope which can be labeled with an antibody.

The polypeptide or peptide which is immobilized on a solid support may be immobilized using an antibody against that polypeptide bound to a solid support or via other technologies which are know *per se*. A preferred *in vitro* interaction may utilize a fusion peptide including glutathione-S-transferase (GST). This may be immobilized on glutathione agarose beads. In an *in vitro* assay format of the type described above a test modulator can be assayed by determining its ability to diminish the amount of labeled peptide or polypeptide (e.g. labeled IL-20Rβ which binds to the immobilized GST-fusion peptide (e.g. immobilized fusion peptide of GST and IL-22R or IL-20Rα). This may be determined by fractionating the glutathione-agarose beads by SDS-polyacrylamide gel electrophoresis. Alternatively, the beads may be rinsed to remove unbound peptide or polypeptide and the amount of peptide or polypeptide which has bound and be determined by counting the amount of label present in, for example, a suitable scintillation counter.

Binding or interaction of two components may also be determined using a two-hybrid assay.

For example, a first polypeptide chain of a receptor herein described may be fused to a DNA binding domain such as that of the yeast transcription factor GAL4. The GAL4 transcription factor includes two functional domains. These domains are the DNA binding domain (GAL4DBD) and the GAL4 transcriptional activation domain (GAL4TAD). By fusing a first polypeptide chain of a receptor to one of those domains, and a second polypeptide chain of the receptor to the respective counterpart, a functional GAL4 transcription factor is restored only when the two polypeptides interact. Thus, interaction of these polypeptides may be measured by the use of a reporter gene linked to a GAL4 DNA binding site which is capable of activating transcription of said reporter gene.

This two hybrid assay format is described by Fields and Song, 1989, Nature 340; 245-246. It can be used in both mammalian cells and in yeast. Other combinations of DNA binding domain and transcriptional activation domain are available in the art and may be preferred, such as the LexA DNA binding domain and the VP60 transcriptional activation domain.

The precise format of any of the screening or assay methods of the present invention may be varied by those of skill in the art using routine skill and knowledge. The skilled person is well aware of the need to employ appropriate control experiments.

The present invention further provides the use of a polypeptide chain of a receptor herein described or peptide fragment thereof, or a receptor as disclosed, in screening for a substance or compound able to affect binding between polypeptide chains of the receptor, or to affect binding between receptor and ligand, or to affect receptor activity.

Performance of an assay method herein described may be followed by isolation and/or manufacture and/or use of a compound, substance or molecule which tests positive for ability to modulate the relevant interaction or affect the relevant biological function or activity. Following identification of a suitable agent, it may be investigated further, and may be modified or derivatized to alter one or more properties, without abolishing its ability to modulate the relevant interaction or affect the relevant biological function. For instance, a single chain Fv antibody molecule may be reformatted into a whole antibody comprising antibody constant regions, e.g. an IgG antibody. Any peptidyl molecule may be modified by addition, substitution, insertion or deletion of one or more amino acids, or by joining of an addition moiety or protein domain. An active agent may be subject to molecular modelling in silico and one or more mimetics of the originally identified agent may be created.

Furthermore, an active agent herein described may be manufactured and/or used in preparation, i.e. manufacture or formulation, of a composition such as a medicament, pharmaceutical composition or drug. These may be administered to individuals.

A compound, whether a peptide, antibody, small molecule or other substance found to have the ability to affect binding between polypeptide chains of a receptor herein described or binding of such a receptor to a ligand has therapeutic and other potential in a number of contexts. For therapeutic treatment such a compound may be used in combination with any other active substance.

Generally, such a substance identified herein and to be subsequently used is provided in an isolated and/or purified form, i.e. substantially pure. This may include being in a composition where it represents at least about 90% active ingredient, more preferably at least about 95%, more preferably at least about 98%. Such a composition may, however, include inert carrier materials or other pharmaceutically and physiologically acceptable excipients. Thus, a composition may consist of the active ingredient obtained using the invention, and an inert carrier. Furthermore, a composition as disclosed may include in addition to an modulator compound as disclosed, one or more other molecules of therapeutic use, such as an anti-tumor agent.

The invention further provides the use of a modulator of a complex of IL-22R and IL-20Rβ in the manufacture of a medicament for the treatment of a subject suffering from a neoplasm or a keratoderma, wherein the modulator of the complex of IL-22R and IL-20Rβ is a soluble form of IL-22R or an antibody against IL-22R.

The invention further provides the use of a modulator of a complex of IL-22R and IL-20Rβ for the manufacture of a medicament for the treatment of a subject suffering from a skin disorder, wherein the modulator comprises a soluble form of IL-22R, provided that when the skin disorder is psoriasis, eczema, atopic or contact dermatitis, said modulator is not a soluble IL-22R/IL-20Rβ heterodimeric polypeptide.

The therapeutic/prophylactic purpose of such uses may relate to any disorder associated with a molecule that binds a receptor of the invention or is associated with an activity or function of a receptor herein described. Disorders that may be treated include disorders of the skin, such as atopic dermatitis, psoriasis, seborrhoeic keratitis, keratodermas and other conditions involving excess proliferation of epidermal and/or skin cells. For example, the mda-7 proteins allegedly has anti-tumor activity because adenoviruses expressing this gene have anti-tumor effects both in vitro and in vivo in mice. Therefore, the results suggest that IL-20 and to some extent, IL-19 have the same activity, suggesting efficacy as antitumor agent.

Also disclosed is a pharmaceutical composition, medicament, drug or other composition for such a purpose, the composition comprising one or more such substances, the use of such a substance in a method of medical treatment, a method comprising administration of such a substance to a patient, e.g. for treatment (which may include preventative treatment) of a medical condition, use of such a substance in the manufacture of a composition, medicament or drug for administration for such a purpose, e.g. for treatment of a medical condition, and a method of making a pharmaceutical composition comprising admixing such a substance with a pharmaceutically acceptable excipient, vehicle or carrier, and optionally other ingredients.

Whatever the substance for the uses be of the present invention, administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the responsibility of general practitioners and other medical doctors.

Pharmaceutical compositions for use in accordance with the present invention, may include, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous or intravenous.

Pharmaceutical compositions for oral administration may be in tablet, capsule, powder inhalation spray (e.g, nebulizers) or liquid form. A tablet may include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required. Examples of techniques and protocols mentioned above can be found in Remington's Pharmaceutical Sciences, 16th edition, Osol, A. (ed), 1980.

Instead of administering such substances directly, they may be produced in target cells by expression from an encoding nucleic acid introduced into the cells, e.g. from a viral vector or as "naked" DNA administered to the body. Nucleic acid encoding the substance e.g. a peptide able to modulate, e.g. interfere with, the interaction of a first and a second polypeptide chain of a receptor herein described, may thus be used in methods of gene therapy, for instance in treatment of individuals, e.g. with the aim of preventing or curing (wholly or partially) a disorder.

The binding properties of receptors herein described further provide for the use of materials and methods for establishing the presence or absence in a test sample, for example obtained from an individual, of particular cytokines or other ligands of such a receptor. For instance, a complex of IL-20Rα and IL-20Rβ binds to all of IL-19, IL-20, and mda-7. Hence, a sample of interest may be contacted with a receptor comprising IL-20Rα and IL-20Rβ, or with cells presenting such a receptor, and it may be determined if any binding occurs. If binding does occur, then one of these cytokines is present. In a second step, the sample may be contacted with a receptor comprising IL-20Rβ and IL-22R, or cells presenting such a receptor. If no binding is detected, this provides indication that IL-19 is present in the sample, but not IL-20 or mda-7. If binding is detected, this provides indication that either or both of mda-7 and IL-20 are present in the sample. Further definition of the sample content can be ascertained by comparing binding of the materials to IL-22R/IL-20Rβ complexes, and IL-20Rα/IL-20Rβ complexes, because the former bind mda-7 better than the latter.

Also described is a method of determining the presence of one or more cytokines in a sample, the method comprising contacting the sample with a receptor of the invention (as disclosed) and determining whether or not the receptor binds to the sample. Preferably, the sample is contacted with one or more combinations of different receptors of the invention in order to identify the cytokine or cytokines present in the sample.

As noted, supra, transgenic mice experiments establish that IL-20 is associated with neonatal lethality, and various skin abnormalities. Hence, one can, e.g., inhibit the effect of IL-20 on receptive cells by contacting the cells with other cytokines which compete for binding with IL-20, or by contacting the receptor with, e.g., an inhibitor of receptor binding, such as an antibody, or some other form of inhibiting molecule. Exemplary of such antagonists or inhibitors are, e.g., mda-7 or IL-19 mutants which have been mutated to abolish activity without abolishing their affinity for the receptor, soluble binding partners for IL-20, such as soluble, extracellular fragments of IL-20 receptors, and so forth.

This mechanism provides an approach to therapies where proliferation of epidermal cells, such as skin cells, is contraindicated. Exemplary of such conditions are atopic dematitis, psoriasis, seborrhoeic keratitis, keratodermas and other conditions involving excess proliferation of epidermal and/or skin cells. In such therapeutic approaches, one administers to a subject in need thereof an amount of an agent which binds to IL-20. Such agents can be any of the inhibitors described supra, including antibodies, portions of antibody molecules which bind to the IL-20 receptor or receptors, fragments of IL-20 itself which are capable of binding to the receptor but do not have effector function, mutated forms of IL-20 which have not lost receptor affinity but are inactive. Such forms of molecules are known for other interleukins, soluble forms of IL-20 receptors, and so forth.

This observation, i.e., that IL-20 stimulates proliferation of epidermal cells, such as skin cells, permits the skilled artisan to develop assays to determine if a test substance of interest has an inhibitory effect on the proliferation of such cells. In effect, one combines the substance of interest with IL-20, and epidermal cells, such as skin cells, determines proliferation, and compares the result to the use of IL-20 in the system alone. A decrease in proliferation indicates an inhibitory effect of the test substance. One can further characterize the specific activity of the test substance by performing parallel experiments using a cell sample, wherein the cells present complexes of IL-22R and IL-20Rβ on their surface, and where complexes of IL-20Rα and IL-20Rβ are presented. For example, if there is inhibition, following stimulation of cells with a test substance and IL-20 and the values are equal, then the inhibitor would probably act on either IL-20 itself, or IL-20Rβ. If the inhibition is greater in the cells presenting IL-22R/IL-20Rβ complexes than those presenting IL-20Rα/IL-20Rβ complexes, then the inhibitor probably acts through IL-22R, and vice versa.

Similarly, the observation that IL-20 responds to complexes of IL-22R and IL-20Rβ suggests methods for enhancing or providing for binding of IL-20 to cells. This can be accomplished, e.g., by transfecting the target cells with nucleic acid molecules encoding IL-20Rβ, in quantities sufficient to form additional, "type II" receptor complexes. As was pointed out, supra, IL-20 & mda-7 are known to have specific effects on cells. One can enhance these effects by, e.g. transforming or transfecting target cells so that they present a greater number of IL-22R and IL-20Rβ. Hence, if the target cells do express IL-22R but not IL-20Rβ, one can transform or transfect the cells with vectors that express IL-20Rβ, to generate additional receptors. Alternatively, one can transform or transfect the cell with nucleic acid molecules encoding both of these molecules, or constructs encoding fusion proteins of the two molecules, one complete molecule and the binding portion of the other, or two binding portions. The relevant portions or molecules could be joined via, e.g., a linker sequence or some other construct which facilitates proper folding and functionality of the molecule.

The results indicate that, by appropriate selection of transfectants, one can "type" samples, such as samples taken from patients, to determine if particular cytokines are present, and to quantify these. For example, it was shown, supra, that complexes of Il-20Rα and IL-20Rβ bind to all of IL-19, IL-20, and mda-7. Hence, in a first part of an assay, one contacts a sample of interest with cells which present complexes of IL-20Rα and IL-20Rβ, and determine if any binding occurs. If binding does occur, then one of these cytokines is present. In a second step, one can then assay the sample with complexes of IL-20Rβ and IL-22R. If the results of this assay are negative, then it can be concluded that IL-19 is present in the sample, but not IL-20 or mda-7.

Similarly, if both assays are positive, one can conclude that either or both of mda-7 and IL-20 are present in the sample Further definition of the sample content can be ascertained by comparing binding of the materials to IL-22R/IL-20Rβ complexes, and IL-20Rα/IL-20Rβ complexes, because the former bind mda-7 better than the latter.

Further aspects and embodiments of the present invention will be apparent to those of ordinary skill in the art.
## Claims

1. Use of a modulator of a complex of IL-22R and IL-20Rβ, for the manufacture of a medicament for the treatment of a subject suffering from a neoplasm or a keratoderma, wherein the modulator of the complex of IL-22R and IL-20R is a soluble form of IL-22R or an antibody against IL-22R.

2. A modulator of a complex of IL-22R and IL-20Rβ for use in the treatment of a subject suffering from a neoplasm or a keratoderma, wherein the modulator of the complex of IL-22R and IL-20Rβ is a soluble form of IL-22R or an antibody against IL-22R.

3. A method for determining if a substance has epidermal cell proliferation inhibition activity, comprising admixing a sample of epidermal cells which present a receptor complex comprising IL-20 Rβ and IL-22R, and a substance to be tested combined with IL-20, measuring epidermal cell proliferation, and comparing said proliferation to proliferation resulting from admixing a sample of said epidermal cells which present said receptor complex with IL-20 alone, a decrease in proliferation being indicative of epidermal cell proliferation inhibition activity of said substance.

4. The method of claim 3, wherein said epidermal cells are skin cells.

5. A method for modulating the effect of interleukin-20 (IL-20) or mda-7 on a cell *in vitro,* comprising contacting said cell with a modulator of a complex of IL-22R and IL-20Rβ, wherein the modulator comprises an isolated soluble form of IL-22R, provided that said modulator is not a soluble IL-22R/IL-20Rβ heterodimeric polypeptide.

6. A method for modulating the effect of interleukin-20 (IL-20) or mda-7 on a skin cell *in vitro,* comprising contacting said cell with a modulator of a complex of IL-22R and IL-20Rβ, wherein the modulator comprises an isolated soluble form of IL-22R.

7. Use of a modulator of a complex of IL-22R and IL-20Rβ for the manufacture of a medicament for the treatment of a subject suffering from a skin disorder, wherein the modulator comprises a soluble form of IL-22R, provided that when said skin disorder is psoriasis, eczema, atopic or contact dermatitis, said modulator is not a soluble IL-22R/IL-20Rβ heterodimeric polypeptide.

8. A modulator of a complex of IL-22R and IL-20Rβ for use in the treatment of a subject suffering from a skin disorder, wherein the modulator comprises a soluble form of IL-22R, provided that when said skin disorder is psoriasis, eczema, atopic or contact dermatitis, said modulator is not a soluble IL-22R/IL-20Rβ heterodimeric polypeptide.

9. The use of claim 7 or the modulator for the use of claim 8 wherein the skin disorder is psoriasis, provided that said modulator is not a soluble IL-22R/IL-20Rβ heterodimeric polypeptide.

10. The use of claim 7 or the modulator for the use of claim 8 wherein the skin disorder is atopic dermatitis, provided that said modulator is not a soluble IL-22R/IL-20Rβ heterodimeric polypeptide.

11. Use of a modulator of a complex of IL-22R and IL-20Rβ for the manufacture of a medicament for the treatment of a subject suffering from seborrhoeic keratitis, wherein the modulator comprises an isolated soluble form of IL-22R.

12. A modulator of a complex of IL-22R and IL-20Rβ for use in the treatment of a subject suffering from seborrhoeic keratitis, wherein the modulator comprises an isolated soluble form of IL-22R.

## Patentansprüche

1. Verwendung eines Modulators eines Komplexes von IL-22R und IL-20Rβ zur Herstellung eines Medikaments zur Behandlung eines Individuums, das an einem Neoplasma oder einem Keratoma leidet, worin der Modulator des Komplexes von IL-22R und IL-20Rβ eine lösliche Form von IL-22R oder ein Antikörper gegen IL-22R ist.

2. Modulator eines Komplexes von IL-22R und IL-20Rβ zur Verwendung zur Behandlung eines Individuums, das an einem Neoplasma oder einem Keratoma leidet, worin der Modulator des Komplexes von IL-22R und IL-20Rβ eine lösliche Form von IL-22R oder ein Antikörper gegen IL-22R ist.

3. Verfahren zur Bestimmung, ob eine Substanz eine die Epidermiszellenproliferation hemmende Aktivität aufweist, umfassend das Kombinieren einer Probe von Epidermiszellen, die einen Rezeptorkomplex präsentieren, der IL-20Rβ und IL-22R umfasst, und einer zu testenden, mit IL-20 kombinierten Substanz, das Messen der Epidermiszellenproliferation und das Vergleichen dieser Proliferation mit der aus der Kombination einer Probe dieser Epidermiszellen, die nur den Rezeptorkomplex mit IL-20 umfasst, resultierenden Proliferation, wobei ein Rückgang der Proliferation ein Indikator für eine die Epidermiszellenproliferation hemmende Aktivität der Substanz ist.

4. Verfahren nach Anspruch 3, worin die Epidermiszellen Hautzellen sind.

5. Verfahren zum Modulieren der Wirkung von Interleukin-20 (IL-20) oder mda-7 auf eine Zelle in vitro, umfassend das Kontaktieren der Zelle mit einem Modulator eines Komplexes von IL-22R und IL-20Rβ, worin der Modulator eine isolierte lösliche Form von IL-22R umfasst, mit der Maßgabe, dass der Modulator kein lösliches heterodimeres IL-22R/IL-20Rβ-Polypeptid ist.

6. Verfahren zum Modulieren der Wirkung von Interleukin-20 (IL-20) oder mda-7 auf eine Hautzelle in vitro, umfassend das Kontaktieren der Zelle mit einem Modulator eines Komplexes von IL-22R und IL-20Rβ, worin der Modulator eine isolierte lösliche Form von IL-22R umfasst.

7. Verwendung eines Modulators eines Komplexes von IL-22R und IL-20Rβ zur Herstellung eines Medikaments zur Behandlung eines Individuums, das an einer Hautkrankheit leidet, worin der Modulator eine lösliche Form von IL-22R umfasst, mit der Maßgabe, dass der Modulator kein lösliches heterodimeres IL-22R/IL-20Rβ-Polypeptid ist, wenn die Hautkrankheit Psoriasis, Ekzem, atopische oder Kontaktdermatitis ist.

8. Modulator eines Komplexes von IL-22R und IL-20Rβ zur Verwendung zur Behandlung eines Individuums, das an einer Hautkrankheit leidet, worin der Modulator eine lösliche Form von IL-22R umfasst, mit der Maßgabe, dass der Modulator kein lösliches heterodimeres IL-22R/IL-20Rβ-Polypeptid ist, wenn die Hautkrankheit Psoriasis, Ekzem, atopische oder Kontaktdermatitis ist.

9. Verwendung nach Anspruch 7 oder Modulator zur Verwendung nach Anspruch 8, worin die Hautkrankheit Psoriasis ist, mit der Maßgabe, dass der Modulator kein lösliches heterodimeres IL-22R/IL-20Rβ-Polypeptid ist.

10. Verwendung nach Anspruch 7 oder Modulator zur Verwendung nach Anspruch 8, worin die Hautkrankheit atopische Dermatitis ist, mit der Maßgabe, dass der Modulator kein lösliches heterodimeres IL-22R/IL-20Rβ-Polypeptid ist.

11. Verwendung eines Modulators eines Komplexes von IL-22R und IL-20Rβ zur Herstellung eines Medikaments zur Behandlung eines Individuums, das an seborrhoischer Keratitis leidet, worin der Modulator eine isolierte lösliche Form von IL-22R umfasst.

12. Modulator eines Komplexes von IL-22R und IL-20Rβ zur Verwendung zur Behandlung eines Individuums, das an seborrhoischer Keratitis leidet, worin der Modulator eine isolierte lösliche Form von IL-22R umfasst.

## Revendications

1. Utilisation d'un modulateur d'un complexe entre l'IL-22R et l'IL-20Rβ, pour la fabrication d'un médicament destiné au traitement d'un sujet souffrant d'un néoplasme ou d'une kératodermie, où le modulateur du complexe entre l'IL-22R et l'IL-20Rβ est une forme soluble de l'IL-22R ou un anticorps dirigé contre l'IL-22R.

2. Modulateur d'un complexe entre l'IL-22R et l'IL-20Rβ, destiné à être utilisé dans le traitement d'un sujet souffrant d'un néoplasme ou d'une kératodermie, où le modulateur du complexe entre l'IL-22R et l'IL-20Rβ est une forme soluble de l'IL-22R ou un anticorps dirigé contre l'IL-22R.

3. Procédé pour déterminer si une substance possède une activité d'inhibition de la prolifération de cellules épidermiques, consistant à mélanger un échantillon de cellules épidermiques, qui présentent un complexe de récepteurs comprenant l'IL-20Rβ et l'IL-22R, avec une substance à tester combinée à l'IL-20, à mesurer la prolifération des cellules épidermiques, et à comparer ladite prolifération à une prolifération observée lorsque l'on mélange un échantillon desdites cellules épidermiques qui présentent ledit complexe de récepteurs avec l'IL-20 seule, une diminution de la prolifération indiquant que ladite substance possède une activité d'inhibition de la prolifération des cellules épidermiques.

4. Procédé selon la revendication 3, dans lequel lesdites cellules épidermiques sont des cellules cutanées.

5. Procédé pour moduler l'effet de l'interleukine-20 (IL-20) ou de mda-7 sur une cellule in vitro, consistant à mettre en contact ladite cellule avec un modulateur d'un complexe entre l'IL-22R et l'IL-20Rβ, où le modulateur comprend une forme soluble isolée de l'IL-22R, à condition que ledit modulateur ne soit pas un polypeptide hétérodimérique IL-22R/IL-20Rβ soluble.

6. Procédé pour moduler l'effet de l'interleukine-20 (IL-20) ou de mda-7 sur une cellule cutanée *in vitro,* consistant à mettre en contact ladite cellule avec un modulateur d'un complexe entre l'IL-22R et l'IL-20Rβ, où le modulateur comprend une forme soluble isolée de l'IL-22R.

7. Utilisation d'un modulateur d'un complexe entre l'IL-22R et l'IL-20Rβ pour la fabrication d'un médicament destiné au traitement d'un sujet souffrant d'une affection cutanée, où le modulateur comprend une forme soluble de l'IL-22R, à condition que lorsque ladite affection cutanée est un psoriasis, de l'eczéma, ou une dermite atopique ou de contact, ledit modulateur ne soit pas un polypeptide hétérodimérique IL-22R/IL-20Rβ soluble.

8. Modulateur d'un complexe entre l'IL-22R et l'IL-20Rβ destiné à être utilisé dans le traitement d'un sujet souffrant d'une affection cutanée, où le modulateur comprend une forme soluble de l'IL-22R, à condition que lorsque ladite affection cutanée est un psoriasis, de l'eczéma, ou une dermite atopique ou de contact, ledit modulateur ne soit pas un polypeptide hétérodimérique IL-22R/IL-20Rβ soluble.

9. Utilisation selon la revendication 7 ou modulateur destiné à une utilisation selon la revendication 8, où l'affection cutanée est un psoriasis, à condition que ledit modulateur ne soit pas un polypeptide hétérodimérique IL-22R/IL-20Rβ soluble.

10. Utilisation selon la revendication 7 ou modulateur destiné à une utilisation selon la revendication 8, où l'affection cutanée est une dermite atopique, à condition que ledit modulateur ne soit pas un polypeptide hétérodimérique IL-22R/IL-20Rβ soluble.

11. Utilisation d'un modulateur d'un complexe entre l'IL-22R et l'IL-20Rβ pour la fabrication d'un médicament destiné au traitement d'un sujet souffrant de kératose séborrhéique, où le modulateur comprend une forme soluble isolée de l'IL-22R.

12. Modulateur d'un complexe entre l'IL-22R et l'IL-20Rβ destiné à être utilisé dans le traitement d'un sujet souffrant de kératose séborrhéique, où le modulateur comprend une forme soluble isolée de l'IL-22R.


<110> Dumoutier, Laure; Renauld, Jean-Christophe
<120> Novel Class Ii Cytokine Receptors and Uses Thereof
<130> LUD 5734.1
<140>
   <141>
<150>US 09/915,735
   <151>2001-07-26
<160> 13
<210> 1
   <211> 39
   <212> DNA
   <213> Artificial sequence
   <220>
   <223> oligonucleotide primer
<400> 1
   gtccttgtag tcacctcccc cgagcttgta gaatttctg 39
<210> 2
   N211> 38
   <212> Dna
   <213> Artificial sequence
   <220>
   <223> oligonucleotide primer
<400> 2
   gtccttgtag tcacctccc cagctgagga catacttc 38
<210> 3
   <211> 39
   <212> DNA
   <213> Artificial sequence
   <220>
   <223> oligonucleotide primer
<400> 3
   gtccttgtag tcacctcccc cttctgtctc ctccatcca 39
<210> 4
   <211> 39
   <212> DNA
   <213> Artificial sequence
   <220>
   <223> oligonucleotide primer
<400> 4
   gtccttgtag tcacctcccc cgacgcaagc atttctcag 39
<210> 5
   <211> 40
   <212> DNA
   <213> Artificial sequence
   <220>
   <223> oligonucleotide primer
<400> 5
   actgctagct cacttgtcgt catcgtcctt gtagtcacct 40
<210> 6
   <211> 29
   <212> DNA
   <213> Artificial sequence
   <220>
   <223> oligonucleotide primer
<400> 6
   ggaggactag ttgccagccc cgatgagga 29
<210> 7
   <211> 31
   <212> DNA
   <213> Artificial sequence
   <220>
   <223> oligonucleotide primer
<400> 7
   gtgtggcggc cgcaggcatg ggattgacag c 31
<210> 8
   <211> 30
   <212> DNA
   <213> Artificial sequence
   <220>
   <223> oligonucleotide primer
   <400> 8
   gccggatcca tgcggccgct gccgctgccg 30
<210> 9
   <211> 31
   <212> DNA
   <213> Artificial sequence
   <220>
   <223> oligonucleotide primer
<400> 9
   atcgctagcc atttagcctt gaactctgat g 31
<210> 10
   <211> 27
   <212> DNA
   <213> Artificial sequence
   <220>
   <223> oligonucleotide primer
<400> 10
   gtggctagcc tggtatgtt tgcccat 27
<210> 11
   <211> 28
   <212> DNA
   <213> Artificial sequence
   <220>
   <223> oligonucleotide primer
<400> 11
   gcgaattcgt ctggcaaaca tttattga 28
<210> 12
   <211> 25
   <212> DNA
   <213> Artificial sequence
   <220>
   <223> oligonucleotide primer
<400> 12
   ttggctagca acaatgttct aggtc 25
<210> 13
   <211> 26
   <212> DNA
   <213> Artificial sequence
   <220>
   <223> oligonucleotide primer
<400> 13
   tgggcgcggc cgcaaaccta tgagat 26